# EUROPEAN PATENT SPECIFICATION

(11) **EP 1 441 705 B1**
(45) Date of publication and mention of the grant of the patent: **03.09.2014**
(21) Application number: 02787578.0
(22) Date of filing: 06.11.2002
(51) Int. Cl.: A61K 9/48, A61K 47/10, A61K 47/14, A61K 9/16

(54) **SOLID, STABILIZED, PROMPT- AND/OR MODIFIED-RELEASE THERAPEUTICAL SYSTEMS FOR THE ORAL ADMINISTRATION OF LIQUID ACTIVE PRINCIPLES, EXCIPIENTS OR FOODSTUFFS**
FESTE STABILISIERTE THERAPEUTISCHE SYSTEME MIT SCHNELLER ODER GESTEUERTER WIRKSTOFFABGABE ZUR ORAL VERABREICHUNG VON FLÜSSIGEN WIRKSTOFFEN, HILFSSTOFFEN ODER LEBENSMITTELN
SYSTEMES THERAPEUTIQUES SOLIDES STABILISES A LIBERATION RAPIDE ET/OU MODIFIEE SERVANT A L'ADMINISTRATION PAR VOIE ORALE DE PRINCIPES ACTIFS, D'EXCIPIENTS OU DE PRODUITS ALIMENTAIRES LIQUIDES

(30) Priority: 09.11.2001 IT MI20012366
(43) Date of publication of application: 04.08.2004
(73) Proprietor: Farmatron Ltd., London W1W 7BL (GB)
(72) Inventor: MASSIRONI, Maria Gabriella, 20125 MILANO (IT)
(74) Representative: Minoja, Fabrizio
(86) International application number: PCT/EP2002/012364
(87) International publication number: WO 2003/039521

(56) References cited:
- EP-A- 0 514 008
- EP-A- 1 116 515
- WO-A-00/26280
- WO-A-00/76478
- WO-A-01/41737
- DE-A- 19 954 923
- US-A- 5 800 834

## Description

The present invention relates to a process for the preparation of liquid active ingredients in solid pharmaceutical, dietetic or alimentary compositions.

The process of the invention comprises adding the liquid active ingredient to a matrix and/or mixture of matrices characterised in that they are solid at room temperature and liquid at temperatures ranging from 30°C to 90°C. Said matrices provide both different release profiles for modulating the *in vitro* and *in vivo* characteristics of medicaments which have to be administered frequently during the day or which have to be released at specific sites of the gastrointestinal tract, as well as giving remarkable stability to the used starting materials, particularly when these are in the liquid form. Active principles, excipients or foodstuffs which are in the liquid form at room temperature can therefore be transformed into the solid form, alone or in combination with other products and/or drugs substances which are known to be poorly stable when formulated in the liquid form and/or that require to be associated in certain combinations.

Formulation of liquid active principles with lipophilic and/or amphipilic matrix systems and other excipients, traditionally used for attaining pharmaceutical formulations with good technological properties, allows to obtain particularly stable solid or semisolid forms, possibly with prompt- or modified- release profiles, adjusting the *in vitro* dissolution rate. Furthermore, amphiphilic and/or lipophilic systems provide the homogeneous distribution of active principles with different chemical-physical characteristics (lipophilic and hydrophilic medicaments) in the formulations.

The resulting matrix is able to stabilize poorly stable products and to modulate constantly and homogeneously the release of the active ingredient, thus obtaining suitable release kinetics.

More particularly, the compositions of the present invention comprise active principles, excipients or foodstuffs belonging to the class of alimentary, dietetic and pharmaceutical oils, alone or in combination with other products.

Examples of ingredients of the compositions of the invention comprise Canola oil, maize oil, cottonseed oil, ethyl oleate, isopropyl myristate, isopropyl palmitate, mineral oil, peanut oil, sesame oil, soybean oil, fish oil, omega 3 fatty acids; enzymes and/or coenzymes such as chymotrypsin, pancreatin, pancrelipase, bromelin, papain, pepsin, coenzyme Q10; carnitines such as L carnitine, acetyl carnitine, propionyl carnitine; liposoluble vitamins such as vitamin E (alpha tocopherol), vitamin D2-D3, vitamin A, vitamin K and various derivatives thereof; active cardiovascular pharmaceutical ingredients such as: Digossine, Methydigossine, Chinidine, Disopiramide, Mexiletine, Propafenone, Flecainide, Amiodarone, Ibopamine, Isosorbide dinitrate, Isosorbide mononitrate, Clonidine, Doxazosin, Urapidil, Cadralazine, Minoxidil, Ketanserine, Hydrochlorothiazide, Chlortalidon, Metolazone, Xipamide, Indapamide, Fenquizone, Furosemide, Bumetamide, Piretamide, Torasemide, Etacrinic acid, Etozoline, Spironolactone, Potassium canreonate, Canrenone, Xanthinol Nicotinate, Pentoxifilline, Nicergoline, Dihydroergocristine, Cyclandelate, Vincamine, Piribedil, Vinburnine, Buflomedil, Naftidrofuril, Pindolol, Propranolol, Timolol, Sotalol, Nadolol, Metoprolol, Atenolol, Acebutol, Betaxolol, Bisoprolol, Celiprolol, Nevibolol, Labetolol, Carvadilol, Amlodipine, Felodipine, Isradipine, Nicardipine, Nifedipine, Nimodipine, Nisoldipine, Nitrendipine, Lacidipine, Manidipine, Lercanidipine, Verapamil, Gallopamil, Diltiazem, Fendiline, Captopril, Enalapril, Lisinopril, Perindopril, Ramipril, Quinapril, Benazepril, Cilazapril, Fosinopril, Trantolapril, Spiropril, Delapril, Moexipril, Zofenopril, Imidapril, Losartan, Eprosartan, Valsartan, Irbesartan, Candesartan, Telmisartan, Simvastatin, Pravastatin, Lovastatin, Fluvastatin, Atorvastatin, Befibrate, Gemfibroxil, Fenofibrate, Colestiramine, Detastrane, Acipimox.

### TECHNOLOGICAL BACKGROUND

Stabilized solid or semisolid formulations from a liquid starting material to obtain forms with different releases can be prepared according to a number of known techniques:
1. Use of inert matrices, in which the main structural component is a material having high surface area, capable of carrying significant amounts of liquids, such as pyrogenic and/or colloidal silicas.
2. Use of hydrophilic matrices, in which the structural component affords a marked resistance to wetting and solubilization in biological fluids, as the system tends to form gels and to gradually swell in time.
3. Use of bioerodible and/or biodegradable matrices, in which the used polymers and materials gradually undergo metabolic and/or physiological degradation at certain biological sites.

All the above mentioned procedures suffer, however, from some drawbacks and disadvantages.

Inert matrices require large amounts of material to obtain a solid product and usually provide non-linear but exponential release kinetics of the active ingredient.

Hydrophilic matrices have at first a linear dissolution profile then, after a certain part of the active ingredient has been released, they deviate from release linearity and often are not able to retain sufficient amounts of liquid active principles.

Bioerodible and/or biodegradable matrices require the ideal enzyme and/or biological environment for the constant release of the drug.

WO 00/76478 discloses controlled-release and taste masking pharmaceutical compositions containing an active ingredient comprising:
- a matrix consisting of lipophilic compounds with melting point lower than 90°C in which the active ingredient is at least partially incorporated;
- optionally an amphiphilic matrix;
- an outer hydrophilic matrix in which the lipophilic matrix and the optional amphiphilic matrix are dispersed.;
- optionally other excipients.

Such compositions contain an inner lipophilic (and optionally amphiphilic) matrix and an outer hydrophilic matrix.

EP 0 514 008 discloses a gastrointestinal mucosa-adherent matrix which is solid at room temperature and which comprises a viscogenic agent that develops viscosity when contacted with water, as dispersed at least in the neighbourhood of the surface layer of a matrix particle containing a polyglycerol fatty acid ester or a lipid and an active ingredient.

US 5,800,834 discloses a method for calculating the optimum quantities of carrier and coating materials required to yield acceptably flowing and compressible liquid/powder admixtures.

### DISCLOSURE OF THE INVENTION

The present invention relates to a process for the preparation of solid or semisolid formulations starting from liquid active principles which after stabilization are released from the system with prompt- or modified- release.

This object has been attained according to the present invention, through the use of amphiphilic and/or lipophilic matrices characterized by melting at temperatures ranging from 30°C to 90°C and being solid at room temperature at least to 25°C. Active principles having pharmacological activity, excipients or foodstuffs in the liquid form can be added to, dissolved or suspended in said melted matrices, to afford solid or semisolid formulations.

Furthermore, amphiphilic and/or lipophilic matrices are suitably selected and formulated for solidifying, stabilizing or suspending appropriate amounts of liquid starting materials and for modulating their release from the system. Moreover, any fast onset phase of the amount of drug present at the surface can be balanced, all the release phases from the system can be homogeneously modulated, including the ability for the formulation to be homogeneously absorbed, without losing the effectiveness of the system.

Upon melting the amphiphilic and/or lipophilic system, the active ingredient is solubilized or dispersed therein, either partially or completely. Cooling to temperatures below 30°C transforms again the system into a semisolid or solid form.

The pharmaceutical composition is suitably distributed in capsules or formulated with other excipients for the preparation of tablets; the active principles or foodstuffs present are highly stabilized and can be released in *vitro*/*in vivo* from the system according to a suitably programmed release profile, which depends on the interaction with the hydrophilic/lipophilic matrix.

Therefore, the invention relates to a process for the formulation of liquid active ingredients in solid pharmaceutical, dietetic or alimentary compositions, which process comprises adding said active ingredients to a melted mass consisting of amphiphilic compounds with melting or softening point ranging from 30 to 60°C and/or lipophilic compounds with melting point ranging from 40 to 90°C, optionally adding any powder excipients or active ingredients and formulating the final compositions.

### DETAILED DISCLOSURE OF THE INVENTION.

The process of the invention comprises the following steps:
a) The amphiphilic/ lipophilic matrix excipients, which can be solid or semisolid, are melted at temperatures above 30°C/90°C, according to the case; or one or more semisolid amphiphilic/ lipophilic excipients are mixed, then melted to obtain a homogeneous solution or dispersion which becomes again semisolid or solid at room temperature.
b) The liquid active ingredient, foodstuff or excipient is solubilized, dispersed or englobated in the matrix from step (a), to obtain a homogeneous solution or dispersion.
c) The liquid system from step (b) can be directly distributed into hard- or soft- gelatin capsules, then left to cool to obtain a semisolid or solid system inside the capsules.
d) The system from step (b) can be added with other solid active pharmaceutical ingredients or excipients with different functions for the preparation of capsules, tablets, granulates, microgranules, sachets, such as silica, cellulose, starches, sugars, polyvinyl pyrrolidones, methacrylates and common glidants, antiaggregants, lubricants such as magnesium stearate, stearic acid, talc.

Amphiphilic compounds for use in the present invention comprise macrogolglycerids consisting of mixtures of mono-, di- and triglycerids and mono- and di- fatty acid esters (gelucire 44/14; gelucire 50/13) mono and diesters, polyethylene glycols hydroxystearates (Solutol HS 15), saccharose monopalmitate (Sucro ester 15), cetostearyl alcohol, cetyl alcohol, non-ionic emulsifying waxes (cetomacrogols).

Lipophilic compounds for use according to the invention comprise mixtures of mono-, di- and triglycerids behenate (compritol "E" ATO) or glyceryl palmitostearate (precirol - biogapress vegetal BM 297 ATO), hydrogenated castor oil, stearic acid, carnauba wax, white wax, yellow wax

These substances can be mixed together, optionally in the presence of an active ingredient, excipient or liquid foodstuff, to obtain different melting or softening points.

The active pharmaceutical ingredient can be englobated in the melted matrix up to a concentration ranging from 0.1 % to 80%.

An alternative procedure for the preparation of a pharmaceutical formulation of the invention comprises granulating the amphiphilic and/or lipophilic matrix by addition of conventional excipients or adjuvants, such as silica, microcrystalline celluloses, starches, lubricants. The matrix is subsequently cooled to obtain a compact, easy-to-process granule or microgranule. An optional dry- or wet- granulation process can be carried out for preparing the final pharmaceutical formulation.

The capsules, microgranules and/or tablets can be subjected to conventional coating processes with gastro-soluble films or be gastro-protected with cellulose and methacrylic polymers.

The active principles which can be conveniently formulated according to the invention comprise:
oily active principles such as canola oil, maize oil, cottonseed oil, ethyl oleate, isopropyl myristate, isopropyl palmitate, mineral oil, peanut oil, sesame oil, soybean oil, fish oil, omega fatty acids, in particular EPA and DHA;
enzymes or co-enzymes such as chymotrypsin, pancreatin, pancrelipase, bromelin, papain, pepsin, coenzyme Q10;
carnitines such as L-carnitine, propionyl carnitine, acetyl carnitine;
Digossine, Methydigossine, Chinidine, Disopiramide, Mexiletine, Propafenone, Flecainide, Amiodarone, Ibopamine, Isosorbide dinitrate, Isosorbide mononitrate, Clonidine, Doxazosin, Urapidil, Cadralazine, Minoxidil, Ketanserine, Hydrochlorothiazide, Chlortalidon, Metolazone, Xipamide, Indapamide, Fenquizone, Furosemide, Bumetamide, Piretamide, Torasemide, Etacrinic acid, Etozoline, Spironolactone, Potassium canreonate, Canrenone, Xanthinol Nicotinate, Pentoxifilline, Nicergoline, Dihydroergocristine, Cyclandelate, Vincamine, Piribedil, Vinburnine, Buflomedil, Naftidrofuril, Pindolol, Propranolol, Timolol, Sotalol, Nadolol, Metoprolol, Atenolol, Acebutol, Betaxolol, Bisoprolol, Celiprolol, Nevibolol, Labetolol, Carvadilol, Amlodipine, Felodipine, Isradipine, Nicardipine, Nifedipine, Nimodipine, Nisoldipine, Nitrendipine, Lacidipine, Manidipine, Lercanidipine, Verapamil, Gallopamil, Diltiazem, Fendiline, Captopril, Enalapril, Lisinopril, Perindopril, Ramipril, Quinapril, Benazepril, Cilazapril, Fosinopril, Trantolapril, Spiropril, Delapril, Moexipril, Zofenopril, Imidapril, Losartan, Eprosartan, Valsartan, Irbesartan, Candesartan, Telmisartan, Simvastatin, Pravastatin, Lovastatin, Fluvastatin, Atorvastatin, Befibrate, Gemfibroxil, Fenofibrate, Colestiramine, Detastrane, Acipimox.
liposoluble vitamins such as vitamin E (alpha tocopherol), vitamin A, vitamin D2-D3, vitamin K and derivatives thereof.

As far as the dissolution characteristics are concerned, these formulations, when contacted with water or aqueous fluids, provide prompt-and/or modified release of the active ingredient which is present in the resulting dispersion, solubilization and/or emulsion of the system.

The following examples illustrate the invention in greater detail.

### EXAMPLE 1

150 g of mono-di-tri glycerides behenate (Compritol or Compritol "E" ATO) are loaded in a melter/homogenizer and heated to about 60°C, that is above their melting point.

The molten mass is added with 500 g of omega 3 fatty acids and/or fish oil and homogenized for some minutes.

The resulting mixture can be distributed while still liquid into soft- or hard- gelatin capsules, in which the mass will solidify once reached room temperature. The average weight content of each capsule is 650 mg.

The capsules were subjected to dissolution test in simulated gastric juices or intestinal environment added with surfactants, showing the following release profile: after 60 minutes no more than 30%, after 180 minutes no more than 60%, after 5 hours no more than 80%.

The resulting product, suitably packaged, shows that the liquid starting material is stabilized.

### EXAMPLE 2

200 g of glyceride palmitoyl stearate (Biogapress Vegetal BM 297 ATO) are loaded in a melter/homogenizer and heated to about 60°C, that is above their melting point. The molten mass is added with 500 g of omega 3 fatty acids and/or fish oil and homogenized for some minutes, then added in succession with 10 g of vitamin E and 10 g of coenzyme Q10 and homogenized. The resulting mixture can be distributed while still liquid into soft- or hard- gelatin capsules, in which the mass will solidify once reached room temperature. The average weight content of each capsule is 720 mg.

The capsules were subjected to dissolution test in simulated gastric juices or intestinal environment added with surfactants, showing the following release profile: after 60 minutes no more than 25%, after 180 minutes no more than 50%, after 5 hours no more than 70%.

The resulting product, suitably packaged, proves to be stabilized.

### EXAMPLE 3

150 g of glyceride palmitoyl stearate (Biogapress Vegetal BM 297 ATO) are loaded in a melter/homogenizer and heated to about 60°C, that is above their melting point. The molten mass is added with 500 g of omega 3 fatty acids and/or fish oil and homogenized for some minutes, then added in succession with 10 g of vitamin E and 100 g of L carnitine or 100 g of acetyl carnitine or 100 g of propionyl carnitine. The resulting mixture can be distributed while still liquid into soft- or hard- gelatin capsules, in which the mass will solidify once reached room temperature. The average weight content of each capsule is 760 mg.

The capsules were subjected to dissolution test in simulated gastric juices or intestinal environment added with surfactants, showing the following release profile: after 60 minutes no more than 30%, after 180 minutes no more than 60%, after 5 hours no more than 80%.

The resulting product, suitably packaged, proves to be stabilized.

### EXAMPLE 4

100 g of mono-, di- and triglycerids and polyethylene glycols and polyglycosylated fatty acids mono and diesters (gelucire 44/14; gelucire 50/14) are loaded in a melter/homogenizer and heated to about 55°C, that is above their melting point. The molten mass is added with 100 g of soy oil and homogenized for some minutes, then added in succession with 10 g of vitamin E or 10 g of vitamin A or 10 g of vitamin D2 or 10 g of vitamin K. The resulting mixture can be distributed while still liquid into soft- or hard- gelatin capsules, in which the mass will solidify once reached room temperature. The average weight content of each capsule is 210 mg.

The capsules were subjected to dissolution test in simulated gastric juices or intestinal environment added with surfactants, showing the following release profile: after 45 minutes more than 70%.

The resulting product, suitably packaged, proves to be stabilized.

### EXAMPLE 5

10 g of coenzyme Q10 are suspended and mixed with 45 g of gelucire 44/14 and 5 g of solutol HS 15 suitably heated to melting temperature and kept at a temperature ranging from 55°C to 65°C. 200 g of microcrystalline cellulose are loaded in a granulator/homogenizer, then the above heated massis added. The components are mixed to granulation and homogeneous dispersion of the matrices, then 20 g of crospovidone, 5 g of magnesium stearate, 5 g of talc and 10 g of colloidal silica are added in succession. The final mixture is tabletted to unitary weight of 300 mg/tablet. The resulting tablets are further film-coated with ethylcellulose and plasticizers.

The tablets were subjected to dissolution test in gastric juices showing the following release profile: after 45 minutes more 70%.

The resulting product, suitably packaged, proves to be stabilized.

### EXAMPLE 6

50 g of gelucire 44/14 are melted and kept at a temperature ranging from 55°C to 65°C. 50 g of glyceride palmitoyl stearate (Precirol) are added to gelucire 44/14 under strong stirring for at least 5 minutes. Said mixture is added with 500 g of fish oil until complete homogenization. 10 g of coenzyme Q10 are loaded in the granulator/melter containing the amphiphilic/lipophilic matrices. The molten mass is placed in a suitable granulator containing 400 g of microcrystalline cellulose, and granulated to obtain a homogeneous mass.

100 g of Prosolv, 5 g of magnesium stearate, 5 g of talc and 10 g of colloidal silica are added in the granulator. The final mixture is tabletted to unitary weight of 1130 mg/tablet. The resulting tablets are then film-coated with ethylcellulose and plasticizers or with polymethacrylates to give a gastro-resistant film.

The tablets were subjected to dissolution test in gastric juices and/or in simulated intestinal environment showing the following release profile: after 60 minutes no more than 25%, after 180 minutes no more than 50%, after 5 hours no more than 70%, after 6 hours no more than 80%.

### EXAMPLE 7

100 g of gelucire 44/14 are melted and kept at a temperature ranging from 55°C to 65°C. 400 g of glyceride palmitoyl stearate (Precirol) are added to gelucire 44/14 under stirring to obtain a homogeneus dispersion. Said mixture is added with 1000 g of omega three triglycerides until complete homogeneization.

40 g of Simvastatine are loaded in the granulator/melter containing the amphiphilic/liphophilic matrices. The molten mass is placed in a suitable granulator containing 500 g of microcrystalline cellulose and 1500 g of maltodextrines.

10 g of magnesium stearate, 10 g of talc, 20 g of colloidal silica and 45 g of flavour are added in the granulator. The final mixture is filled into sachets to unitary weight of 3625 mg/sachet. The sachets were subjected to dissolution test in gastric juices and/or in simulated intestinal environment showing the following release profile for Simvastatin: after 60 minutes no more than 30%; after 180 minutes non more than 50%; after 5 hours no more than 70%; after 6 hours no more than 90%.

## Claims

1. A process for the formulation of liquid active ingredients in solid pharmaceutical, dietetic or alimentary compositions, which comprises:
a) melting a mass consisting of solid or semisolid amphiphilic compounds with melting or softening point ranging from 30 to 60°C and/or solid or semisolid lipophilic compounds with melting point ranging from 40 to 90°C, to obtain a homogeneous solution or dispersion which becomes again solid or semisolid at room temperature;
b) dispersing the liquid active ingredient in the molten matrix, to obtain a homogeneous solution or dispersion;
c) optionally adding to the homogeneous solution or dispersion obtained in b) other solid pharmaceutical ingredients or excipients;
d) cooling the homogeneous solution or dispersion to obtain a solid or semisolid system.

2. A process as claimed in claim 1 wherein the active ingredients are selected from vegetable or animals oils and liposoluble vitamins.

3. A process as claimed in claim 2 wherein the active ingredients are selected from fish oil, soybean oil, maize oil, cottonseed oil, peanut oil, sesame oil, Canola oil, vitamin E, vitamin K.

4. A process as claimed in any one of claims 1-3 wherein the amphiphilic compounds are selected from macrogolglycerids consisting of mixtures of mono-, di- and triglycerids and polyethylene glycols mono and diesters and polyglycosylated fatty acids, hydroxystearate polyethylene glycols, saccharose monopalmitate, cetostearyl alcohol, cetyl alcohol, non-ionic emulsifying waxes (cetomacrogols).

5. A process as claimed in any one of claims 1-3 wherein the lipophilic compounds are selected from mono-, di- and triglycerids behenate or glyceryl palmitostearate, hydrogenated castor oil, stearic acid, carnauba wax, white wax, yellow wax.

6. A process as claimed in any one of claims 1-5 wherein the liquid active ingredient is added to a molten mass consisting of only amphiphilic compounds.

7. A process as claimed in any one of claims 1-5 in which the liquid active ingredient is added to a molten mass consisting of only lipophilic compounds.

8. A process as claimed in any one of claims 1-5 wherein the active liquid ingredient is added to a molten mass consisting of a mixture of amphiphilic compounds and lipophilic compounds.

9. A process as claimed in any one of claims 1 to 8, wherein the molten mass is further added with powder, solid or semi-solid active pharmaceutical ingredients or excipients selected from enzymes, coenzyme Q10, vitamins, carnitine and derivatives, starches, silica, microcrystalline celluloses, lubricants.

10. A process as claimed in any one of claims 1-9 wherein the final formulation is obtained by cooling the molten mass into hard- or soft- gelatin capsules.

11. A process as claimed in any one of claims 1-9 wherein the final formulation is obtained by granulation optionally followed by tabletting or distribution of the granulate into the dosage form.

12. A process as claimed in any one of claims 1 to 11, wherein the molten mass is further added with solid active pharmaceutical ingredients selected from Digossine, Methydigossine, Chinidine, Disopiramide, Mexiletine, Propafenone, Flecainide, Amiodarone, Ibopamine, Isosorbide dinitrate, Isosorbide mononitrate, Clonidine, Doxazosin, Urapidil, Cadralazine, Minoxidil, Ketanserine, Hydrochlorothiazide, Chlortalidon, Metolazone, Xipamide, Indapamide, Fenquizone, Furosemide, Bumetamide, Piretamide, Torasemide, Etacrinic acid, Etozoline, Spironolactone, Potassium canreonate, Canrenone, Xanthinol Nicotinate, Pentoxifilline, Nicergoline, Dihydroergocristine, Cyclandelate, Vincamine, Piribedil, Vinburnine, Buflomedil, Naftidrofuril, Pindolol, Propranolol, Timolol, Sotalol, Nadolol, Metoprolol, Atenolol, Acebutol, Betaxolol, Bisoprolol, Celiprolol, Nevibolol, Labetolol, Carvadilol, Amlodipine, Felodipine, Isradipine, Nicardipine, Nifedipine, Nimodipine, Nisoldipine, Nitrendipine, Lacidipine, Manidipine, Lercanidipine, Verapamil, Gallopamil, Diltiazem, Fendiline, Captopril, Enalapril, Lisinopril, Perindopril, Ramipril, Quinapril, Benazepril, Cilazapril, Fosinopril, Trantolapril, Spiropril, Delapril, Moexipril, Zofenopril, Imidapril, Losartan, Eprosartan, Valsartan, Irbesartan, Candesartan, Telmisartan, Simvastatin, Pravastatin, Lovastatin, Fluvastatin, Atorvastatin, Befibrate, Gemfibroxil, Fenofibrate, Colestiramine, Detastrane, Acipimox.

## Patentansprüche

1. Verfahren zur Formulierung von flüssigen aktiven Bestandteilen in feste pharmazeutische, diätetische oder Nahrungszusammensetzungen, das umfasst:
a) Schmelzen einer Masse, die aus festen oder halbfesten amphiphilen Verbindungen mit einem Schmelz- oder Erweichungspunkt, der von 30 bis 60°C reicht, und/oder festen oder halbfesten lipophilen Verbindungen mit einem Schmelzpunkt, der von 40 bis 90°C reicht, besteht, um eine homogene Lösung oder Dispersion zu erhalten, die bei Raumtemperatur wieder fest oder halbfest wird,
b) Dispergieren des flüssigen aktiven Bestandteils in der geschmolzenen Matrix, um eine homogene Lösung oder Dispersion zu erhalten,
c) gegebenenfalls Zusetzen von anderen festen pharmazeutischen Bestandteilen oder Exzipienzien zu der in b) erhaltenen homogenen Lösung oder Dispersion,
d) Abkühlen der homogenen Lösung oder Dispersion, um ein festes oder halbfestes System zu erhalten.

2. Verfahren, wie in Anspruch 1 beansprucht, wobei die aktiven Bestandteile aus pflanzlichen oder tierischen Ölen und fettlöslichen Vitaminen ausgewählt werden.

3. Verfahren, wie in Anspruch 2 beansprucht, wobei die aktiven Bestandteile aus Fischöl, Sojabohnenöl, Maisöl, Baumwollsamenöl, Erdnussöl, Sesamöl, Rapsöl, Vitamin E, Vitamin K ausgewählt werden.

4. Verfahren, wie in einem jeglichen der Ansprüche 1-3 beansprucht, wobei die amphiphilen Verbindungen aus Macrogolglyceriden, bestehend aus Gemischen von Mono-, Di- und Triglyceriden und Polyethylenglykolmono- und -diestern und polyglycosylierten Fettsäuren, Hydroxystearatpolyethylenglykolen, Saccharosemonopalmitat, Cetostearylalkohol, Cetylalkohol, nichtionischen emulgierenden Wachsen (Cetomacrogole) ausgewählt werden.

5. Verfahren, wie in einem jeglichen der Ansprüche 1-3 beansprucht, wobei die lipophilen Verbindungen aus Mono-, Di- und Triglyceridbehenaten oder Glycerylpalmitostearat, hydriertem Rizinusöl, Stearinsäure, Carnaubawachs, Paraffin, Gelbwachs ausgewählt werden.

6. Verfahren, wie in einem jeglichen der Ansprüche 1-5 beansprucht, wobei der flüssige aktive Bestandteil zu einer geschmolzenen Masse, die aus lediglich amphiphilen Verbindungen besteht, zugegeben wird.

7. Verfahren, wie in einem jeglichen der Ansprüche 1-5 beansprucht, bei dem der flüssige aktive Bestandteil zu einer geschmolzenen Masse, die aus lediglich lipophilen Verbindungen besteht, zugegeben wird.

8. Verfahren, wie in einem jeglichen der Ansprüche 1-5 beansprucht, wobei der flüssige aktive Bestandteil zu einer geschmolzenen Masse, die aus einem Gemisch von amphiphilen Verbindungen und lipophilen Verbindungen besteht, zugegeben wird.

9. Verfahren, wie in einem jeglichen der Ansprüche 1 bis 8 beansprucht, wobei die geschmolzene Masse ferner mit pulverförmigen, festen oder halbfesten aktiven pharmazeutischen Bestandteilen oder Exzipienzien, ausgewählt aus Enzymen, Coenzym Q10, Vitaminen, Carnitin und Derivaten, Stärken, Silica, mikrokristallinen Cellulosen, Schmiermitteln, versetzt wird.

10. Verfahren, wie in einem jeglichen der Ansprüche 1-9 beansprucht, wobei die Endformulierung durch Abkühlen der geschmolzenen Masse in Hart- oder Weichgelatinekapseln erhalten wird.

11. Verfahren, wie in einem jeglichen der Ansprüche 1-9 beansprucht, wobei die Endformulierung durch Granulierung, gegebenenfalls gefolgt von Tablettierung oder Verteilung des Granulats in die Dosisform, erhalten wird.

12. Verfahren, wie in einem jeglichen der Ansprüche 1 bis 11 beansprucht, wobei die geschmolzene Masse ferner mit festen aktiven pharmazeutischen Bestandteilen, ausgewählt aus Digossin, Methydigossin, Chinidin, Disopiramid, Mexiletin, Propafenon, Flecainid, Amiodaron, Ibopamin, Isosorbiddinitrat, Isosorbidmononitrat, Clonidin, Doxazosin, Urapidil, Cadralazin, Minoxidil, Ketanserin, Hydrochlorothiazid, Chlortalidon, Metolazon, Xipamid, Indapamid, Fenchizon, Furosemid, Bumetamid, Piretamid, Torasemid, Etacrinsäure, Etozolin, Spironolacton, Kaliumcanreonat, Canrenon, Xanthinolnicotinat, Pentoxifillin, Nicergolin, Dihydroergocristin, Cyclandelat, Vincamin, Piribedil, Vinburnin, Buflomedil, Naftidrofuril, Pindolol, Propranolol, Timolol, Sotalol, Nadolol, Metoprolol, Atenolol, Acebutol, Betaxolol, Bisoprolol, Celiprolol, Nevibolol, Labetolol, Carvadilol, Amlodipin, Felodipin, Isradipin, Nicardipin, Nifedipin, Nimodipin, Nisoldipin, Nitrendipin, Lacidipin, Manidipin, Lercanidipin, Verapamil, Gallopamil, Diltiazem, Fendilin, Captopril, Enalapril, Lisinopril, Perindopril, Ramipril, Quinapril, Benazepril, Cilazapril, Fosinopril, Trantolapril, Spiropril, Delapril, Moexipril, Zofenopril, Imidapril, Losartan, Eprosartan, Valsartan, Irbesartan, Candesartan, Telmisartan, Simvastatin, Pravastatin, Lovastatin, Fluvastatin, Atorvastatin, Befibrat, Gemfibroxil, Fenofibrat, Colestiramin, Detastran, Acipimox, versetzt wird.

## Revendications

1. Procédé de formulation d'ingrédients actifs liquides dans des compositions pharmaceutiques, diététiques ou alimentaires, solides, qui comprend les étapes consistant à :
a) faire fondre une masse constituée de composés amphiphiles solides ou semi-solides à point de fusion ou de ramollissement allant de 30 à 60 °C et/ou de composés lipophiles solides ou semi-solides à point de fusion allant de 40 à 90 °C, pour obtenir une solution ou dispersion homogène qui redevient solide ou semi-solide à température ambiante ;
b) disperser l'ingrédient actif liquide dans la matrice fondue, pour obtenir une solution ou dispersion homogène ;
c) facultativement, ajouter à la solution ou dispersion homogène obtenue en b) d'autres ingrédients pharmaceutiques ou excipients solides ;
d) refroidir la solution ou dispersion homogène pour obtenir un système solide ou semi-solide.

2. Procédé selon la revendication 1 dans lequel les ingrédients actifs sont choisis parmi les huiles végétales ou animales et les vitamines liposolubles.

3. Procédé selon la revendication 2 dans lequel les ingrédients actifs sont choisis parmi l'huile de poisson, l'huile de soja, l'huile de maïs, l'huile de coton, l'huile d'arachide, l'huile de sésame, l'huile de canola, la vitamine E, la vitamine K.

4. Procédé selon l'une quelconque des revendications 1 à 3 dans lequel les composés amphiphiles sont choisis parmi les macrogolglycérides constitués de mélanges de mono-, di- et triglycérides et de mono et diesters de polyéthylèneglycols et d'acides gras polyglycosylés, les hydroxystéarates de polyéthylèneglycols, le monopalmitate de saccharose, l'alcool cétostéarylique, l'alcool cétylique, les cires émulsifiantes non ioniques (cétomacrogols).

5. Procédé selon l'une quelconque des revendications 1 à 3 dans lequel les composés lipophiles sont choisis parmi les béhénates de mono-, di-et triglycéryle ou le palmitostéarate de glycéryle, l'huile de ricin hydrogénée, l'acide stéarique, la cire de carnauba, la cire blanche, la cire jaune.

6. Procédé selon l'une quelconque des revendications 1 à 5 dans lequel l'ingrédient actif liquide est ajouté à une masse fondue constituée uniquement de composés amphiphiles.

7. Procédé selon l'une quelconque des revendications 1 à 5 dans lequel l'ingrédient actif liquide est ajouté à une masse fondue constituée uniquement de composés lipophiles.

8. Procédé selon l'une quelconque des revendications 1 à 5 dans lequel l'ingrédient liquide actif est ajouté à une masse fondue constituée d'un mélange de composés amphiphiles et de composés lipophiles.

9. Procédé selon l'une quelconque des revendications 1 à 8, dans lequel la masse fondue est en outre additionnée d'ingrédients pharmaceutiques actifs ou d'excipients pulvérulents, solides ou semi-solides, choisis parmi les enzymes, le coenzyme Q10, les vitamines, la carnitine et ses dérivés, les amidons, la silice, les celluloses microcristallines, les lubrifiants.

10. Procédé selon l'une quelconque des revendications 1 à 9 dans lequel la formulation finale est obtenue par refroidissement de la masse fondue pour former des capsules de gélatine dures ou molles.

11. Procédé selon l'une quelconque des revendications 1 à 9 dans lequel la formulation finale est obtenue par granulation facultativement suivie par un compactage en comprimé ou une distribution du granulat dans la forme posologique.

12. Procédé selon l'une quelconque des revendications 1 à 11, dans lequel la masse fondue est en outre additionnée d'ingrédients pharmaceutiques actifs solides choisis parmi les suivants : digoxine, méthyldigoxine, quinidine, disopyramide, mexilétine, propafénone, flécaïnide, amiodarone, ibopamine, dinitrate d'isosorbide, mononitrate d'isosorbide, clonidine, doxazosine, urapidil, cadralazine, minoxidil, kétansérine, hydrochlorothiazide, chlortalidone, métolazone, xipamide, indapamide, fenquizone, furosémide, bumétamide, pirétamide, torasémide, acide étacrinique, étozoline, spironolactone, canréonate de potassium, canrénone, nicotinate de xanthinol, pentoxifylline, nicergoline, dihydroergocristine, cyclandélate, vincamine, piribédil, vinburnine, buflomédil, naftidrofuril, pindolol, propranolol, timolol, sotalol, nadolol, métoprolol, aténolol, acébutol, bétaxolol, bisoprolol, céliprolol, névibolol, labétolol, carvadilol, amlodipine, félodipine, isradipine, nicardipine, nifédipine, nimodipine, nisoldipine, nitrendipine, lacidipine, manidipine, lercanidipine, vérapamil, gallopamil, diltiazem, fendiline, captopril, énalapril, linisopril, périndopril, ramipril, quinapril, bénazépril, cilazapril, fosinopril, trantolapril, spiropril, delapril, moexipril, zofénopril, imidapril, losartan, éprosartan, valsartan, irbésartan, candésartan, telmisartan, simvastatine, pravastatine, lovastatine, fluvastatine, atorvastatine, béfibrate, gemfibroxil, fénofibrate, colestyramine, détastrane, acipimox.
